(19) Europäisches Patentamt

European Patent Office

Office européen des brevets

(11) Publication number: **0 255 387**
A2

## (12) EUROPEAN PATENT APPLICATION

(21) Application number: **87306765.6**

(22) Date of filing: **30.07.87**

(51) Int. Cl.³: **A 62 B 18/02**
**A 61 M 16/06**

(30) Priority: **31.07.86 GB 8618707**

(43) Date of publication of application:
**03.02.88 Bulletin 88/5**

(84) Designated Contracting States:
**AT BE CH DE FR LI LU NL SE**

(71) Applicant: Haycock, John Francis
5 Pinfold Lane
Scarisbrick near Ormskirk Lancashire L40 8HR(GB)

(72) Inventor: Haycock, John Francis
5 Pinfold Lane
Scarisbrick near Ormskirk Lancashire L40 8HR(GB)

(74) Representative: Huntingford, David Ian et al,
W.P. THOMPSON & CO. Coopers Building Church Street
Liverpool L1 3AB(GB)

(54) **Respiratory device.**

(57) A respiratory device for use by people with breathing problems or in cold weather, is in the form of a face mask (10) having a heat exchanging portion (14). The heat exchanging portion (14) comprises a metal honeycomb (20) sandwiched between two layers (22) of spun polypropylene, and has an air inlet aperture (16).

As a wearer exhales, the warm breath warms up the metal honeycomb (20) which transfers its heat to the air drawn in by subsequent inhalations. After only a few exhalations and inhalations, the inhaled air is found to be warm. The spun polypropylene layers (22) cause turbulence in the inhaled and exhaled air, and help the air to be drawn through substantially all of the channels (21) in the honeycomb.

Fig 2.

EP 0 255 387 A2

## DESCRIPTION

### RESPIRATORY DEVICE.

The present invention relates to a respiratory device, and in particular, but not exclusively, to a device for aiding the respiration of patients and/or for aiding respiration in cold weather.

Certain medical conditions, for example angina and asthma, are aggravated if the sufferer inhales air which is significantly lower than body temperature. In such cases, it is necessary in cold weather for a sufferer to remain indoors in a heated room to avoid aggravating the condition. This is obviously very inconvenient, since periods of cold weather can last for weeks and, in many countries, months.

It is also desirable for people who are recovering from a heart attack to breathe air which is at a temperature close to body temperature. It has been found to be much easier for the patient than breathing air at the ambient temperature.

The problem of inhaling cold air also exists for people who work in the open during cold weather, for example, workers on oil rigs. Such workers often find it very uncomfortable to breathe air at a low temperature when, in certain climates, their bodies may be 50° Centigrade or more warmer than the air which they are inhaling. Such people normally attempt to overcome the problem by wrapping a scarf or other garment around their mouths during cold weather, but it is found that in order to achieve the desired effect, several layers of material have to be used, which restricts the flow of air to the wearer's mouth, which is undesirable if a person is working manually, when a relatively free flow of air into the worker's lungs is desirable.

It is an object of the present invention to provide a respiratory device which allows a user to inhale air which is at a greater temperature than the ambient temperature, while at the same time, allowing a relatively free flow of air through the device.

In accordance with the present invention, there is provided a respiratory device characterised by an air inlet, an air outlet, and heat exchanging means interposed between the inlet and the outlet.

Preferably, the heat exchanging means comprises material having a plurality of air passages therethrough.

The heat exchanging means may comprise material which increases the flow path of air through the device, or may comprise material which does not substantially effect the flow path.

There may be means for causing turbulence in the air which is to pass through the heat exchanging means, e.g. a layer of aerated material, such as polypropylene.

In a preferred embodiment, the heat exchanging means comprises metal, e.g. aluminium, which is provided with a plurality of passages therethrough. In a preferred embodiment, the aluminium is in the form of a honeycomb, preferably with the apertures aligned substantially in the direction in which air would flow if the honeycomb were not present. A suitable example of such honeycomb is the aluminium honeycomb manufactured by the American Cyanamid Co., which is used in aircraft construction and which has hexagonal honeycombs whose opposite faces are spaced apart by approximately 1/8 of an inch (0.3175 cm). The heat exchanging means may be made from other materials, such as paper, metal wool or plastics, e.g. spun polypropylene.

In one embodiment, the respiratory device is incorporated in a face mask, and in a second embodiment, the respiratory device may be self-contained and may have an attachment for fitting to the aperture of a conventional face mask.

By way of example only, specific embodiments of the present invention will now be described, with reference to the accompanying drawings, in which:-

-4-

Fig.1 is a perspective view of a first embodiment of respiratory device according to the present invention;

Fig.2 is a cross-sectional side elevation of the respiratory device illustrated in Fig.1; and

Fig.3 is a perspective view of an alternative embodiment of respiratory device according to the present invention.

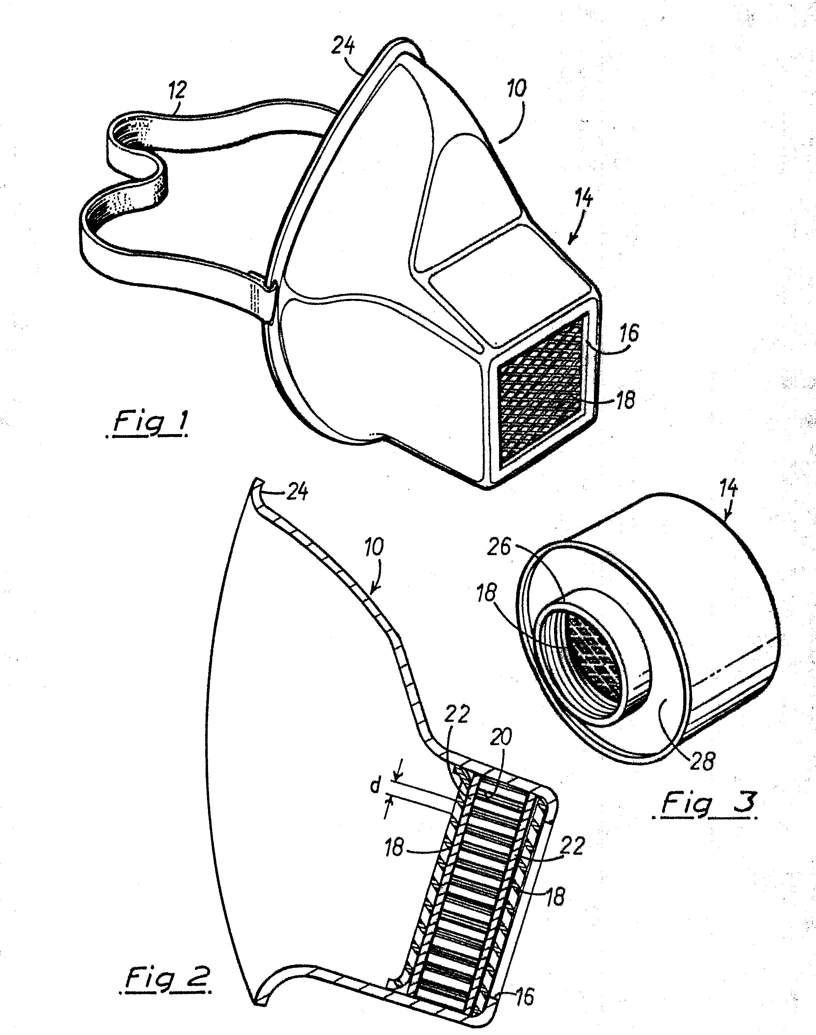

Referring to Figs. 1 and 2, an embodiment of heat exchanger according to the present invention comprises a mask 10 which is made from a soft, resilient plastics, e.g. polypropylene, which is shaped to fit over the nose and mouth of a patient, and which is provided with an elastic strap 12 to allow it to be positioned securely on a patient's face. The front of the mask is shaped into a substantially cuboidal heat exchanging portion 14 which is provided with a square aperture 16 at its front.

As best seen in Fig.2, the heat exchanging portion 14 is hollow. Postioned at either end of the heat exchanging portion 14 are aluminium grilles 18 which, as will be explained, enclose the heat exchanging means. Positioned within the heat exchanging portion 14 is an aluminium hexagonal honeycomb structure 20, whose channels 21 are aligned substantially perpendicular to the planes of the aluminium grilles 18. In the example given, the distance between opposite faces of the honeycomb is approximately 1/8 inch (0.3175 cm), although the actual dimensions of the honeycomb may be varied as required, as will be explained hereinafter. At either end of the honeycomb 20 is positioned a layer 22 of spun polypropylene which, as will be explained, causes air turbulence through the heat exchanger.

In use, the mask is fitted over a wearer's face, and the resilient nature of the mask causes a flanged edge 24 of the mask to abut the face substantially sealingly. The mask is held in position on the face by positioning the strap 12 on a wearer's head. The wearer breathes normally through the mask, but because of the good seal around the edge of the mask, substantially all air which is inhaled and exhaled must pass through the heat exchanging portion 14.

When a patient exhales air, the air is warm (approximately body temperature). This exhaled air passes through the grille 18 inside the mask 10, and passes through the first polypropylene layer 22 which induces turbulence in the air, and causes it to pass through substantially all the hexagonal channels 21 in the honeycomb portion 20. The metal honeycomb portion 20 absorbs heat from the exhaled air which cools down and is expelled through the second polypropylene layer 22 and the grille 18. When air is inhaled by a wearer, it passes through the aperture 16 and the outer grille 18 and through the outer spun polypropylene 22, where it becomes turbulent, causing it to pass through substantially all of the hexagonal channels 21 of the honeycomb portion 20. The incoming air heats up as it passes through the channels 21 in the honeycomb member which have become heated due to the passage of exhaled air therethrough. By the time the inhaled air reaches the inner end of the channels 21 in the honeycomb portion 20, it is substantially warmer than air outside the mask, such that the air actually inhaled by the wearer of the mask is warm.

In use, it is found that only about a dozen exhalations and inhalations are required to heat up the honeycomb portion sufficiently to warm up incoming air to an acceptable temperature. Thus it can be seen that when it is desired to use the mask, it may be brought into operation very quickly. If it is desired to use the mask out-of-doors in cold weather, it is advisable to take a few breaths indoors before going outside, since in this way the heat exchanger will warm up much more quickly than if the first few breaths are with cold air from outside.

An alternative embodiment of the present invention is illustrated in Fig.3 and is in the form of a cylindrical heat exchanging box 14' which is not integrally connected to a mask, but which is provided with an internally threaded connection 26 in one face of the heat exchanger 14' by means of which the heat exchanger may be connected to a conventional face mask which is usually provided with a hole in the front portion to which various filters may be attached. By means of connecting a tube between the hole in a mask and the threaded portion 26 on the heat exchanging portion, it is possible to adapt a conventional mask to work according to the present invention. The construction of the second embodiment would be generally similar to the heat exchanging portion 14 of the first embodiment, with the exceptions that the portion 14' is cylindrical and that an end wall 28 is formed at the end remote from the apertured end 16, the wall being slightly spaced apart from the innermost gauze, to allow air to be inhaled through all the honeycomb channels 21. The construction of the second embodiment would otherwise be identical to the heat exchanging portion of the first embodiment.

It will be seen that the embodiments described will allow a wearer to breathe warm air, and that the large cross-sectional area of the heat exchanging portions 14,14' and the many passages therethrough allow a relatively free, uninterrupted flow of air therethrough.

The invention is not restricted to the details of the foregoing embodiments. For example, the honeycomb portion 20 may be dispensed with, and may be replaced with any other material which absorbs heat from warm gases passing thereover. Examples of suitable alternatives are metal wool, paper and plastics. Moreover, the heat exchanger could be filled with layers of spun polypropylene only, which would provide a very cheap alternative.

The present invention has the advantage that the flow of air through the heat exchanging portion is hardly restricted, thus allowing virtually free and normal breathing by a wearer. The device also has the effect of retaining moisture in the air by condensing the moisutre which would normally be lost into the atmosphere. This feature is of importance when the device is used during inhalation of anaesthetic gases, and oxygen, as it reduces the drying effect of the said compressed gases when inhaled. This is especially beneficial to patients in intensive care after heart attacks, who require moist, warm, oxygenated air to breathe.

.........................................................

## CLAIMS

1. A respiratory device characterised by an air inlet (16), an air outlet and heat exchanging means (20) interposed between the inlet (16) and the outlet.

2. A respiratory device as claimed in claim 1, wherein the heat exchanging means (20) comprises material having a plurality of air passages therethrough.

3. A respiratory device as claimed in claim 1 or claim 2, wherein the heat exchanging means (20) comprises material (22) which increases the flow path of air through the device.

4. A respiratory device as claimed in any of claims 1 to 3, comprising means (22) for causing turbulence in the air which is to pass through the heat exchanging means.

5. A respiratory device as claimed in claim 4, wherein the heat exchanging means (20) is positioned between two means (22) for causing turbulence in the air.

6. A respiratory device as claimed in any of claims 1 to 5, wherein the heat exchanging means (20) comprises a metal formation with a plurality of passages (21) therethrough.

7. A respiratory device as claimed in claim 6, wherein the metal defines a plurality of aligned passages (21).

8. A respiratory device as claimed in claim 7, wherein the passages (21) are aligned substantially in the direction in which air would flow if the metal were not present.

9. A respiratory device as claimed in any of claims 1 to 8, wherein the device is incorporated in a face mask (10).

10. A respiratory device as claimed in any of claims 1 to 8, wherein the device is self-contained and is provided with means (26) for affixing it to an aperture of a face mask.

_Fig 1_

_Fig 2_

_Fig 3_